# EUROPEAN PATENT SPECIFICATION

(11) **EP 3 115 055 B1**
(45) Date of publication and mention of the grant of the patent: **29.08.2018**
(21) Application number: 15460092.8
(22) Date of filing: 02.10.2015
(51) Int. Cl.: A61K 36/53, A61P 17/02

(54) **HERBAL PREPARATION COMPRISING AN EXTRACT OF MELITTIS MELISSOPHYLLUM FOR USE IN WOUND HEALING**
KRÄUTERPRÄPARAT ENTHALTEND EIN EXTRAKT AUS MELITTIS MELISSOPHYLLUM ZUR VERWENDUNG IN DER WUNDBEHANDLUNG
PRÉPARATION COMPRENANT UN EXTRAIT DE MELITTIS MELISSOPHYLLUM POUR LE TRAITMENT DES BLESSURES

(30) Priority: 09.07.2015 PL 41307415
(43) Date of publication of application: 11.01.2017
(73) Proprietor: Wyzsza Szkola Medyczna, 15-875 Bialystok (PL)
(72) Inventor: Tomulewicz, Mikolaj, 15-507 Bialystok (PL)

(56) References cited:
- SLAVICA GRUJIC ET AL: "Evaluation of antioxidant activity of Melittis melissophyllum L. extracts", ARHIV ZA BIOLOSKE NAUKE, vol. 66, no. 4, 1 January 2014 (2014-01-01), pages 1401-1410, XP55303112, ISSN: 0354-4664, DOI: 10.2298/ABS1404401G
- BILJANA KAURINOVIC ET AL: "Antioxidant Activities of Melittis melissophyllum L. (Lamiaceae)", MOLECULES, vol. 16, no. 12, 14 April 2011 (2011-04-14), pages 3152-3167, XP55303237, DOI: 10.3390/molecules16043152
- Anonymous: "Herbs-Treat and Taste: BASTARD BALM - A HERB WITH MANY TRADITIONAL MEDICINAL USES: HISTORY AND POSSIBLE HEALTH BENEFITS OF BASTARD BALM", , 22 November 2011 (2011-11-22), XP55303229, Retrieved from the Internet: URL:http://herbs-treatandtaste.blogspot.de /2011/11/bastard-balm-herb-with-many-tradi tional.html [retrieved on 2016-09-16]

## Description

The invention relates to a herbal preparation for wound healing with anti-inflammatory and astringent actions used in the herbal medicine to treat conditions related to the interruption of the anatomical continuity of the outer layers of the skin or deeper tissues.

Herbal preparations are widely used in herbal medicine, cosmetics and conventional medicine. Its effect is due to the presence of biological active substances, which act after application on the skin. Superficial skin inflammation are very serious problem. Untreated or inadequately treated can be a cause of the infection. The healing process is painful and often limited mobility and quality of life. An important and still current problem in medicine is the rate of healing of various wounds - both caused by mechanical factors (abrasions, lacerations, cuts) as well as thermal or chemical (burns), as well as a side effect of different diseases - such as diabetes and the related problem of diabetic foot.

All significant injuries caused vascular damage and interruption of their continuity and thus initiate molecular and cellular response leading to the activation of the hemostasis process. The healing process can not be initiate, until hemostatic mechanisms not work, which are the multifactorial and multistep process. The most important element of hemostasis is blood clotting, leading to the clot formation. The clot consists mainly of gird of the fibrin and embedded on them platelets. The clot formation is very important process. It prevents further loss of fluids and electrolytes from the wound and reduces pollutions from the outside.

The healing process of the skin can be accelerated by the action of all kinds of chemical substances mainly of synthetic origin, which often produce additional side effects mainly affecting the weakening elasticity damaging to proteins in the skin.

The current treatment of this type disorders is generally used ointment with steroids, and with more chronic conditions strong antibiotics is used, which often cause adverse side effects.

Another way of treatment skin injuries is used eg. Calendula ointment, which contains calendula and Vaseline. When we used this ointment, however, the healing process is slow and the crust formed on the wound surface can be easily produced microorganisms. For the treatment of skin inflammation, including wound, can be also apply special herbal compositions with synergic mechanism of action.

From the international patent WO 9742963 and American patent US 5 061 491 are known herbal compositions used to treat wounds and burns. They contain in its compositions, among others, plantain, chamomile and calendula, yarrow, eucalyptus, celandine.

From the Polish patent P. 198268 is known a herbal composition for the treatment of chronic wounds, particularly used in venous ulcers and postphlebitic syndrome, which contains a mixture of herbs consisting of marigold flower, herb firefly plantain, Chamomile, Echinacea, wherein the composition comprises the active ingredients in the form of carotenoids, iridoids, aucubin, catalpol and polyphenols, particularly flavonoid and phenolic acids in an amount resulting from the ratio contained in the composition of plants. From the articles of Slavica M. Grujić et al.: "Evaluation of antioxidant activity of Melittis melissophyllum L. extracts." and Biljana Kaurinovic et al.: "Antioxidant activities of Melittis melissophyllum L. (Lamiacae)." there has been known method of measure antioxidant activity of flavonoids and polyphenols presented in alcoholic extract of Melittis melissophyllum herb. Cited above articles concerning only the antioxidant impact of plant components (flavonoids and polyphenols) on the pro-oxidative activity some enzymes. Surprisingly, it was found that the extract of melissian honey extract (Melittis melissophyllum) accelerates the wound healing process, which is accompanied by an increase in the amount and phagocytic activity of neutrophils from the surface of the healing wound. In the world becoming clearer the trend of seeking natural substances that may replace the synthetic one.

The aim of the invention is widening of the medical preparations based on herbal extract for the treatment of conditions associated with interruption of the anatomical continuity of the outer layers of the skin or deeper tissues, which would allow the healing process and to allow to obtain the desired results even in the treatment of chronic and inflammatory complications after surgery.

Surprisingly, the herbal preparation, according to the invention, is equal pharmacological formulations with a bactericidal or bacteriostatic properties, which has exacerbated therapeutic effect leading to a rapid self-cleaning skin wounds with interruption the anatomical continuity of the outer layers or deeper tissues and complete their healing without causing side effects.

The herbal preparation for the wound healing, according to the invention, comprising, as an active substance, alcoholic extract of plant emulsified or suspended in an organic medium is characterized in that it contains a *Melittis melissophyllum* herb in an amount from 10% to 40% w/w, ethyl alcohol in an amount from 10% to 20% w/w.

The herbal preparation for the wound healing in the form of the ointment contains as an organic medium Vaselinum album in an amount from 40% to 70% w/w.

There is preferably, when the herbal preparation for the wound healing in the form of the ointment contains glycerol or propylene glycol in an amount of 2% w/w, trimethylamine in an amount of 2% w/w, hydroxycellulose in an amount of 1% w/w and aqua purifficata in an amount from 30% to 35% w/w.

Another aspect of the invention is the use of the herbal preparation for the wound healing to treat various types of wounds and skin inflammations.

The herbal preparation for the wound healing, according to the invention, shows normal epithelial growth and accelerates granulation. It allows to maintain longest moisture of the wound environment, which accelerates their filling, wherein due to the long duration occurred the destruction of the connective tissue and subcutaneous tissue and growth epidermis.

The herbal preparation for the wound healing based on the herbal extract from melittis - *Melittis melissophyllum* L. contains active substances such as tannins, polyphenols flavonoids, amine compounds, bitter substances and mineral salts

The studies showed, primarily, large amounts of tannins which have astringent properties and the ability to create especially with collagen insoluble and irreversible connections which are not subject to putrefaction. In addition, they are active astringently for mucous membranes preventing hemorrhage from the capillary blood vessels, they also inactivate bacteria and their toxins, and they are also have anti-inflammatory properties. Otherwise, the presence of the flavonoids as a means of sealing the walls of small blood vessels have been used as anti-bleeding compounds, preventing ecchymosis and varicose veins. Their activity is associated with inhibition of enzymes present in the vessel walls - hyaluronidase which is responsible for the degradation one of the intracellular substances and increase of permeability of the intracellular spaces. It also have been shown that they have anti-aggregation potential on platelets.

The inclusion of the Melittis melissophyllum L. to the cultivation allowed to get the standardized raw material with specified quality parameters form one side, but on the other hand they allowed to increase in the population of this plant in their natural environment, thus reducing the risk of their extinction.

The present invention will be described in examples with reference to certain preferred solution, which does not limit other solutions/embodiments according to the invention.

The herbal preparation contains the extract from the Melittis melissophyllum L. was used to treat wounds of the epidermis and deeper layers of skin, in which the inflammatory processes roll on.

### Example I

The following semi-sold formulation was prepared to the study obtain by mixing together:
- 40% w/w Melittis melissophyllum L. from the whole plant,
- 20% w/w 96% ethyl alcohol,
- 40% w/w Vaselinum album (hydrocarbon medium used in lipophilic ointments).

Etched by the ethyl alcohol substance is dissolved with heating in the smooth ointment medium in order to its better dispersion (a biologically active compounds contained in the test extract). It could be mixed also in cold lipophilic medium. It is important that the substance has been dissolved in the medium at the concentration below saturation.

In the study was involved three groups of animals with open wounds:
I control group - it was not apply any treatment on the wound (even antiseptic).
II group - it was used typical antiseptic agent - chlorhexidine (according with the standard protocol of the treatment of infected wounds)
III group - it was used the preparation with Melittis melissophyllum L. extract.

The phagocytic activity of neutrophils was assessed using phagocytic index (PI) - the percentage of phagocytic containing adsorbed latex particles, and an amount of phagocytes (IF) - the average number of molecules per phagocyte.

The results of the effect of the application of alcohol extract of *Melittis melissophyllum L.* during epithelialization (epidermal) and wound healing in rats showed in Table 1.

The results of determining the level of phagocytic activity of leukocytes in healing of the wound surface after application of the alcohol extract of *Melittis melissophyllum L.* mammal showed in Table 2.

**Table 1.**

| | Gropu | | |
|---|---|---|---|
| | control | Chlorhexidine | *Melittis melissophyllum L.* |
| Beginning epitelialization, 24-hrs | 5,0±0,4 | 3,9±0,4 | 3,5±0,2 |
| Beginning rejection of the crust, 24-hrs | 9,0±0,5 | 7,6±0,5 | 6,5±0,4 |
| Full crust rejection, 24-hrs | 11,5±0,3 | 10,5±0,3 | 7,8±0,5 |
| Complete wound healing, 24-hrs | 13,00±0,91 | 11,25±0,19 | 9,3±0,7 |

**Table 2.**

| Group | Time after granulation | | | | | |
|---|---|---|---|---|---|---|
| | 3 day | | 5 day | | 7 day | |
| | Phagocytic Index (PI), % | The amount of phagocytes (IF) | PI,% | IF | PI,% | IF |
| Control | 54,60± 1,12 | 7,0±1,2 | 82,0±2,28 | 11,0±2,5 | 66,20±4,16 | 12,5±3,0 |
| Chlorhexidine | 61,60±9,49 | 10,0±2,5 | 59,01±4,58 | 14,0±3,0 | 48,20±5,51 | 11,0±3,0 |
| *Melittis melissophyllum L.* | 82,40±1,40 | 16,0±3,5 | 89,00±0,45 | 22,0±2,8 | 41,20±3,50^{a} | 9,0±1,5 |
| PI - = FI - phagocytic index - the percentage of phagocytes containing latex particles. | | | | | | |
| IF - = FF, the amount of phagocytes - the average numer of particles per 1 phagocyte. | | | | | | |

It was found that extract from *Melittis melissophyllum L.* accelerates wounds healing in experimental group of animals compared to control group. The first, region crust rejection in the third group was starting at 6-7 days, in the control groups (saline, chlorhexidine) - 9 and 7-8 days, respectively. The final crust rejection in the third group was 7-8 day, in control group - 10-11 days. Animals receiving herb extract from *Melittis melissophyllum L.* were complete epithelialization of the wound surface at 9-th day, while the control group, in which on the wound surface the saline solution or chlorhexidine was applied, the full epithelialization of wound surface was observed at 12-13-th day.

The phagocytic activity of neutrophils from the wound surface in different periods of wound healing (increase in phagocytic indecs and number of phagocytes) due to *Melittis melissophyllum L.* extract was shown in Table 2.

It was found the first time, that the *Melittis melissophyllum L.* extract has a high therapeutic activity during wound healing period in all stages of wound healing, accelerating wound repair process and cell proliferation and therefore provide complete wound healing much earlier.

In the mechanism of action *Melittis melissophyllum L.* extract are probably involved vasodilation and normalization of microcirculation disorders, improving tissue metabolism, increased phagocytosis function of neutrophils and macrophage activation, directly or indirectly affect the proliferation of fibroblasts and angiogenesis.

### Example II

It was prepared the herbal preparation consisting of:
- 40% w/w alcoholic extract of *Melittis melissophyllum L.* obtained from the whole plant by etching dry rough by 96% ethyl alcohol,
- 20% w/w 96%ethyl alcohol,
- glycerol in an amount of 2%w/w,
- triethyloamnie in an amount of 2% w/w,
- hydroxycellulose in an amount of 1% w/w,
- aqua purifficata in an amount of 35% w/w.

Formulation above was used in the treatment of open wounds. It was used as described in example I.

It was found that the herbal preparation based on *Melittis melissophyllum L.* definitely accelerates the repair processes during wound healing. Complete wound healing was significantly earlier.

## Claims

1. Herbal preparation for use in wound healing containing as an active substance an alcoholic plant extract emulsified or suspended in an organic medium, wherein the extract contains *melittis melissophyllum* herb in an amount from 10% to 40% w/w, and ethyl alcohol in an amount from 10% to 20% w/w.

2. Herbal preparation for use as in claim 1 **characterized in that** the organic medium contains Vaselinum album in an amount from 40% to 70% w/w.

3. Herbal preparation for use as in claim 1 **characterized in that** the organic medium contains glycerol or propylene glycol in an amount of 2% w/w, trimethylamine in an amount of 2% w/w, hydroxycellulose in an amount of 1% w/w, aqua purifficata in an amount from 30 to 35 % w/w.

4. Herbal preparation for use as in claim 1 wherein the wound is caused by damage to the skin or mucous membranes.

## Patentansprüche

1. Das Kräuterpräparat, das zur Heilung der Wunden genutzt wird, es enthält den Wirkstoff alkoholischer Pflanzenextrakt emulgiert oder aufgehängt im organischen Boden, in dem der Extrakt die Pflanze *mellitis melissophyllum* von 10% bis 40% Gewicht und Äthylalkohol in der Menge von 10% bis 20% Gewicht enthält.

2. Das Kräuterpräparat zur Anwendung nach Anpruch 1, ist **dadurch gekennzeichnet, dass** der organische Boden Vaselinum album in der Menge von 40% bis 70% Gewicht enthält.

3. Das Kräuterpräparat zur Anwendung nach Anpruch 1, ist **dadurch gekennzeichnet, dass** der organische Boden Glycerol oder Propylenglykol in der Menge von 2 % Gewicht, Trietylamin in der Menge von 2 % Gewicht, Hydroxycellulose in der Menge von 1 Gewicht, aqua purifficata in der Menge von 30% bis 35% Gewicht enthält.

4. Das Kräuterpräparat zur Anwendung nach Anpruch 1, in dem die Wunde durch eine Verletzung der Kontinuität der Haut oder der Schleimhaut verursacht ist.

## Revendications

1. Préparation à base de plantes pour la cicatrisation contenant comme substance active un extrait végétal alcoolique émulsifié ou en suspension dans un milieu organique, dans lequel l'extrait contient de l'herbe de *mellitis melissophyllum* en une quantité de 10% à 40% de poids et de l'alcool éthylique en une quantité de 10% à 20% de poids.

2. Préparation à base de plantes pour une utilisation selon la restriction n.1, **caractérisée en ce que** le milieu organique contient du Vaselinum album en une quantité de 40% à 70% de poids.

3. Préparation à base de plantes pour une utilisation selon la restriction n.1, **caractérisée en ce que** le milieu organique contient du glycérol ou du propylèneglycol en une quantité de 2% de poids, de la triméthylamine en une quantité de 2% de poids, de l'hydroxycellulose en une quantité de 1% de poids, de l'eau ultra pure en une quantité de 30 à 35% de poids.

4. Préparation à base de plantes pour une utilisation selon la restriction n.1, dans laquelle la blessure est provoquée par une lésion de la peau ou des membranes muqueuses.
